# EUROPÄISCHE PATENTSCHRIFT

(11) **EP 2 190 945 B1**
(45) Veröffentlichungstag und Bekanntmachung des Hinweises auf die Patenterteilung: **29.02.2012**
(21) Anmeldenummer: 08834044.3
(22) Anmeldetag: 26.09.2008
(51) Int. Cl.: C09K 11/06

(54) **ORGANISCHES STRAHLUNGSEMITTIERENDES BAUTEIL**
ORGANIC RADIATION-EMITTING COMPONENT
COMPOSANT ORGANIQUE À ÉMISSION DE RAYONNEMENT

(30) Priorität: 28.09.2007 DE 102007046445; 15.01.2008 DE 102008004471; 25.01.2008 DE 102008006113; 27.03.2008 DE 102008015940; 20.05.2008 WO PCT/DE2008/000868
(43) Veröffentlichungstag der Anmeldung: 02.06.2010
(73) Patentinhaber: OSRAM Opto Semiconductors GmbH, 93055 Regensburg (DE)
(72) Erfinder: SCHMID, Günter, 91334 Hemhofen (DE)
(74) Vertreter: Epping - Hermann - Fischer
(86) Internationale Anmeldenummer: PCT/DE2008/001588
(87) Internationale Veröffentlichungsnummer: WO 2009/039845

(56) Entgegenhaltungen:
- WO-A-2006/008976
- US-A1- 2006 222 887
- US-A1- 2006 240 282
- US-A1- 2007 048 546
- BERRY J F; COTTON F A; HUANG P; MURILLO C A; WANG X: "A hardwon dirhodium paddlewheel with guanidinate type (hpp) bridging ligands" DALTON TRANSACTIONS, 7. Dezember 2005 (2005-12-07), Seiten 3713-3715, XP002512869

## Beschreibung

Die vorliegende Erfindung betrifft ein organisches strahlungsemittierendes Bauteil wie z.B. eine Organische Leuchtdiode (OLED), das zumindest zwei Elektrodenschichten und dazwischen zumindest eine organische strahlungsemittierende Schicht mit einem Triplettemitter umfasst sowie eine hierfür geeignete phosphoreszente Metallkomplexverbindung.

Diese Patentanmeldung beansprucht die Priorität der deutschen Patentanmeldungen 10 2007 046 445.4, 10 2008 015 940.9, 10 2008 006 113.1 und 10 2008 004 471.7, deren Offenbarungsgehalt hiermit durch Rückbezug aufgenommen wird. Diese Patentanmeldung beansprucht weiterhin die Priorität der internationalen Patentanmeldung PCT/DE2008/000868, deren Offenbarungsgehalt bezüglich Komplexen mit Guanidin-Anionen oder Komplexen mit Liganden, die eine anionische Guanidingruppe enthalten, hiermit durch Rückbezug aufgenommen wird.

Der Stand der Technik stellt eine Vielzahl von OLEDs bereit, die rot und/oder grün leuchten. OLEDs, die tiefblau, hellblau und/oder blaugrün strahlenden und annehmbare, weil wirtschaftlich interessantere, Lebensdauern haben sind seltener.

Cotton et al. haben gezeigt, dass der hpp-Ligand (Anion des 1,3,4,6,7,8-hexahydro-2H-pyrimido[1,2-a]pyrimidine = Hhpp) die außerordentliche Fähigkeit besitzt, Komplexe in hohen Oxidationsstufen durch seine enorme Basisizät zu stabilisieren (F. A. Cotton, L. M. Daniels, C.A. Murillo, D.J. Timmons, C.C. Wilkinson, J. Am. Chem. Soc. 2002, 124, 9249-9256, sowie Tab. 3 aus F. A. Cotton, N. E. Gruhn, J. Gu, P. Huang, D. L. Lichtenberger, C. A. Murillo, L. 0. van Dorn, C. C. wilkinson; "Closed-Shell Molecules That Ionize More Readily Than Cesium", Science Vol 298 (2002) 1971. ).

Die Druckschrift US 2006/0240282 Al betrifft ein Emissionsmaterial und ein organisches elektrolumineszierendes Bauelement mit dem Emissionsmaterial. Die Druckschrift US 2007/0048546 Al betrifft ein organisches phosphoreszentes Licht emittierendes Bauelement, in der Druckschrift US 2006/0222887 Al wird ein organisches elektrolumineszierendes Bauelement angegeben. In der Veröffentlichung Berry et al. ("A hardwon dirhodium paddlewheel with guanidinate type (hpp) bridging ligands", Dalton Trans., 2005, p. 3713-3715) wird eine Dirhodium Paddlewheel Komponente angegeben.

Aufgabe der vorliegenden Erfindung ist es, eine verbesserte OLED zur Verfügung zu stellen. Diese Aufgabe wird durch ein strahlungsemittierendes organisches Bauteil gemäß Anspruch 1 und durch eine phosphoreszente Metallkomplexverbindung gemäß Anspruch 10 gelöst.

Es wird ein strahlungsemittierendes organisches Bauteil angegeben, aufweisend ein Substrat, zumindest eine untere Elektrodenschicht, zumindest eine organische strahlungsemittierende Schicht und darüber zumindest eine obere Elektrodenschicht, wobei in der emittierenden Schicht in einer Matrix zumindest ein strahlungsemittierender Metallkomplex enthalten ist, der zumindest einen Liganden, der über eine Guanidin-Anion-Gruppe am Zentralatom koordiniert ist, aufweist. Nachfolgend wird ein derartiger Ligand, der ein Guanidin-Anion ist oder eine anionische Guanidingruppe enthält auch Guanidinat-Ligand genannt.

Vorteilhafterweise enthält der in der Matrix in der Emissionsschicht eingebettete Metallkomplex zumindest einen anionischen Liganden, der die Struktureinheit eines Anions, abgeleitet vom Guandin, also der Guanidin-Anion-Gruppe: 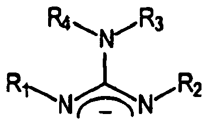 enthält. Dieser Guanidinat-Ligand kann substituiert oder unsubstituiert sein.

Weitere Metallkomplexe mit niedrigen Ionisierungsenthalpien sind auch aus dem oben zitierten Science-Artikel von Cotton et al. bekannt. Diese Metallkomplexe wurden bislang zur n-Dotierung von organischen halbleitenden Materialien eingesetzt. In der folgenden Tabelle, die aus dem genannten Artikel von Cotton et al. entnommen ist, sind ausgewählte Beispiele derartiger Metallkomplexe, an denen der so genannte "hpp-" Ligand, ein "paddlewheel"-Ligand gebunden ist, dargestellt.

**Table 3. First ionizations of atoms and chemically prepared and isolated molecules below 5 cV. Transient and low-temperature species detected in molecular beam techniques are discussed in the text. Atl of the dinuclear molecules have closed-shell singtet σ²π⁴δ² configurations.**

| Molecule or atom | Spin state | IE (eV) | Type | Ref. |
|---|---|---|---|---|
| W₂(hpp)₄ | | 3.514 | Onset | This work |
| W₂(hpp)₄ | | 3.76 | Vertical | Thls work |
| Cs | Doublet | 3.89 | Atomlc | (3, 22, 23) |
| (16-C6 Ey (η⁵-C₅Me₅)Fe | Doublet | 3.95 | Onset | (23) |
| Mo₂(hpp)₄ | | 4.01 | Onset | This work |
| Fr | Doublet | 4.07 | Atomic | (21, 23) |
| Rb | Doublet | 4.18 | Atomic | (3, 22, 23) |
| (η⁶-C₆Et)(η⁵-C₅Me₅)Fe | Doublet | 4.21 | Vertical | (23) |
| Mo₂(hpp)₄ | | 4.33 | Vertical | This work |
| K | Doublet | 4.34 | Atomic | (3,12, 23) |
| Cp(η⁶-C₆Et)Fe | Doublet | 4.54 | Vertical | (24) |
| Cp(η⁶-C₆Me₆)Fe | Doublet | 4.68 | Vertical | (24) |
| (η⁵-C₅Me₅)₂Co | Doublet | 4.71 | Vertical | (25) |
| Cp(η⁶-C₆H₃(CMe₃)₃)Fe | Doublet | 4.74 | Vertical | (24) |
| Cr₂(hpp)₄ | | 4.76 | Onset | This work |
| (η⁵-C₉Me₇)₂Co | Doublet | 4.89 | Vertical | (26) |
| (η⁵-C₅Me₅)₂Cr | Triplet | 4.93 | Vertical | (25) |
| Cr₂(hpp)₄ | | 5.00 | Vertical | This work |

Überraschend hat sich gezeigt, dass Metallkomplexe mit dem dort offenbarten "hpp"-Ligand und auch generell Metallkomplexe mit Guanidinat-Liganden in Emittersystemen oder Emitterschichten für organische Leuchtdioden zu einer effizienten kurzwelligen Emission führen und dabei auch eine ausreichende Stabilität zeigen. Die Emitter eignen sich zum Beispiel zur Emission von roter Strahlung, oranger Strahlung, gelber Strahlung, grüner Strahlung, blauer Strahlung und violetter Strahlung. Zu nennen sind insbesondere auch Emitter, die tiefblau (kleiner etwa 450 nm), hellblau (etwa 450 nm bis 500 nm) und/oder blaugrün (größer etwa 500 nm) emittieren. In den Metallkomplexen können dabei neben den genannten Guanidinat-Liganden auch ein oder mehrere (gleiche oder verschiedene) weitere Liganden (im folgenden Koliganden genannt) enthalten sein.

Vorteilhafterweise umfasst der Metallkomplex ein Übergangsmetallatom oder ein Lanthanoid als Zentralatom, insbesondere ein Übergangsmetall der 7., 8., 9., 10., oder 11. Gruppe des Periodensystems, bevorzugt Ir, Pt, Au, Re, Rh, Ru, Os, Pd, Ag, Zn; besonders bevorzugt ist Iridium, Platin und Gold. Dabei kann auch mehr als ein substituierter oder unsubstituierter Guanidinat-Ligand gebunden sein.

Nachfogend werden die erfindungsgemäß möglichen Bindungsmöglichkeiten des Guanidinat-Liganden an den Metallzentren beispielhaft mit einem hpp-Liganden gezeigt. Der Ligand kann dabei nur an ein Metallzentrum koordiniert sein oder verbrückend wirken. Im Sinne der Erfindung sind auch gemischte Varianten, wo ein hpp-Ligand zweizähnig an ein erstes Metallatom gebunden ist, während ein weiterer an dem ersten Metallatom und einem zweiten Metallatom verbrückend wirkt.

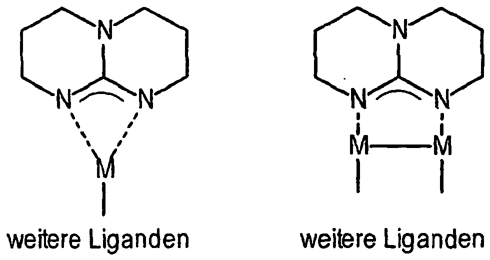

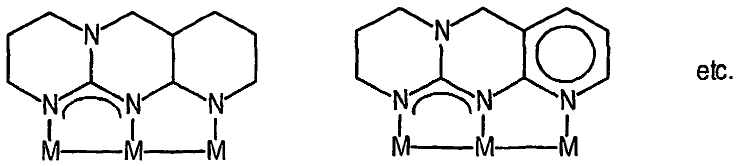

Die Koordinationssphäre des Metallatoms wird gegebenenfalls durch weitere beliebige Koliganden, insbesondere auch Liganden, abgeleitet vom Guandingerüst vervollständigt.

Gemäß vorliegender Erfindung haben sich nicht nur Komplexe mit dem hpp-Ligand selbst in Emissionsschichten strahlungsemittierender Bauteile als vorteilhaft erwiesen, sondern insbesondere auch Liganden mit einem in vielfältiger Weise modifizierten Guandin-Grundgerüst.

Bislang wurden die Guanidinat-Liganden nicht für Emittersysteme benutzt, weil ein wissenschaftliches Vorurteil bestand, die nicht voll durchkonjugierten Liganden, wie die mit Guanidin-Gruppe, würden sich für Emittersysteme nicht eignen. Unter einem voll durchkonjugierten Liganden wird hierbei ein Ligand verstanden, der mindestens einen Aromaten und/oder mehrere konjugierte Doppelbindungen enthält.

Erfindungsgemäß wurde hingegen festgestellt, dass Guanidinat-Liganden zur Stabilisierung in metallorganischen phosphoreszenten Emittern herangezogen werden können, obwohl in diesen kein durchkonjugiertes System vorliegt. Die Emissionswellenlänge der erfindungsgemäßen Guanidinat-Komplexe kann daher auch vom Koliganden bestimmt sein. Weiterhin wurde festgestellt, dass der Guanidinat-Ligand den emittierenden Komplex gegenüber Elektronen stabilisiert. Die Guanidinat-Liganden können dabei weitere Substituenten enthalten, die die Koordination an weitere Metallatome fortsetzen. Dann entstehen mehrkernige Komplexe.

Somit enthält der in der Matrix der Emissionsschicht eingebettete Metallkomplex zumindest einen anionischen Liganden mit der allgemeinen Strukturformel:

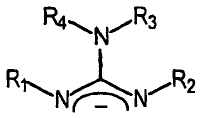

Durch Variation der der Reste R₁, R₂, R₃ und R₄ lassen sich verschiedenartige Liganden erzeugen, die geeignet für die Emittersysteme im erfindungsgemäßen Bauteil sind. R₁, R₂, R₃ und R₄ können dabei unabhängig voneinander H, unverzweigte (z.B. Methyl-, Ethyl-), verzweigte, kondensierte (wie z.B. Decahydronaphthyl-) und ringförmige (wie z. B. Cyclohexyl-) Alkylreste, Aromaten, kondensierte Aromaten, Heterocyclen und kondensierte Heterocyclen sowie gegebenenfalls vollständig oder teilweise substituierte Alkylreste, Aromaten, kondensierte Aromaten, Heterocyclen und kondensierte Heterocyclen sein.
Weiterhin können die Gruppen R₁ und R₄ und/oder die Gruppen R₂ und R₃ (und gegebenenfalls auch die Gruppen R₃ und R₄) miteinander verbunden sein, und insbesondere eine Alkylen-Brücke darstellen, so das ein Ring ausgebildet wird, der insbesondere 5- oder 6-gliedrig sein kann. Die Alkylreste und Alkylenreste können Ethergruppen (Ethoxy-, Methoxy-, Propoxy-, usw.), Ester-, Amid-, Carbonatgruppen etc. enthalten. R₁, R₂, R₃ und R₄ sind - wie erwähnt - nicht auf gesättigte Systeme beschränkt, sondern können auch folgende Reste beinhalten bzw. hierin bestehen: substituierte bzw. unsubstituierte Aromaten und Heterocyclen. Als Aromaten sind hierbei insbesondere zu nennen: Phenyl, Diphenyl, Naphthyl, Phenanthryl etc. bzw. Benzyl etc. Eine Zusammenstellung in Frage kommender Heterocyclen ist nachfolgend dargestellt:

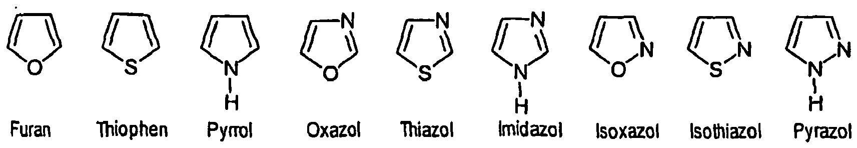

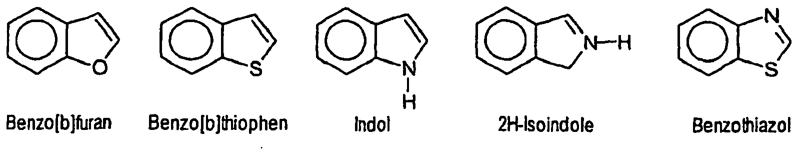

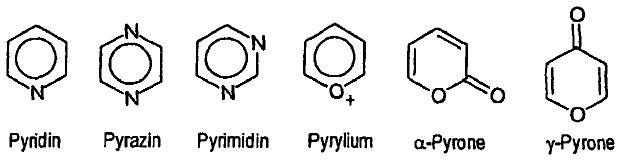

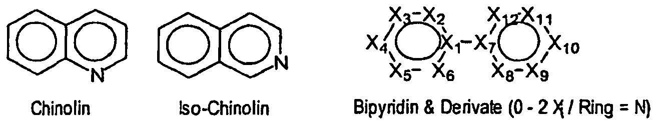

Dies ist nur eine Auswahl von substituierten bzw, unsubstituierten Heterozyklen, die als Reste R₁, R₂, R₃ und R₄ oder als Bestandteil dieser Reste in Frage kommen. Der Einfachheit halber ist nur die Grundeinheit dargestellt. Die Bindung an den Liganden kann an jeder bindungsfähigen Stelle des Grundkörpers oder einen Linker erfolgen. Zudem können diese Reste selbst noch beispielweise durch elektronenziehende oder e-lektronschiebende Gruppen substituiert sein.

Als Koliganden kommen alle Liganden und Ligandensysteme in Frage, die bislang in Komplexen, die als Emitter in organischen selbstemittierenden Bauteile wie beispielsweise in OLEDs (organischen Leuchtdioden) verwendet wurden oder als dazu geeignet beschrieben wurden, in Betracht. Grundsätzlich eignen sich im Wesentlichen einzähnige, zweizähnige oder mehrzähnige über ein C-, N-, P-, As-, Sb-, O-, S- und/oder Se-Atom an das Zentralatom koordinierte Koliganden. Einige bekannte Beispiele für Koliganden finden sich beispielsweise in WO2005097942A1, WO2006013738A1, WO2006098120A1, WO2006008976A1, WO2005097943A1 oder, US 6,902,830, US 7,001,536, US 6,830,828. Als Koliganden eignen sich daher erfindungsgemäß insbesondere eine Vielzahl von zumindest zweizähnigen Liganden, die über ein C-Atom und ein N-Atom oder über zwei N-Atome an das Metallatom koordiniert vorliegen (Beispiel: 2-Phenylpyridin oder 2-Phenylimidazol)zum Einsatz in Emittern für organische Leuchtdioden.

Als Koliganden sind auch Liganden geeignet, die über eine Fluorierung der Phenylpyridin-Liganden in Bis(2,4-difluorophenyl-2-pyridyl)-Iridium(III)-picolinat (FIrPic) oder Bis(2,4-difluorophenylpyridinato)-tetrakis(1-pyrazolyl)borate-Iridium(III) (FIr6) erhalten werden. Die Fluorierung verschiebt die Emission zu kürzeren Wellenlängen. weiterhin geeignet sind Carbenliganden (WO200519373 oder EP1692244B1); durch Erhöhung der Elektronendichte wird hiereine elektronische Struktur erzeugt, die eine tiefblaue Emission erzeugt.

Der Inhalt der vorstehend in Bezug auf die Koliganden genannten Druckschriften, insbesondere was Struktur und Synthese der Koliganden betrifft, soll hiermit per Referenz Bestandteil der Offenbarung der vorliegenden Beschreibung sein. Alle Verbindungen in den genannten Patenten sind in diese Erfindungsmeldung einbezogen, sofern sie die beschriebenen Bedingungen durch die beanspruchte Modifikation erfüllen.

Im Unterschied zu Metallkomplexen, die ausschließlich Koliganden enthalten, weisen die erfindungsgemäßen Komplexe mit Liganden, die über eine darin enthaltene Guanidin-Anion-Gruppe an das Zentralatom koordiniert sind, eine verbesserte Stabilität und eine leichtere Zugänglichkeit auf.

Bevorzugt wird durch die Liganden, die über eine darin enthaltene Guanidin-Anion-Gruppe an das Zentralatom koordiniert sind, in homo- bzw. heteroleptischen organometallischen Komplexen zumindest ein Zentralatom insbesondere in der Oxidationsstufe Ir(III), Pt(II) und/oder Au(I) stabilisiert, wobei das Emissionspektrum des Komplexes - sofern die vorstehend angegebenen Koliganden enthalten sind - zur kürzeren Emissionswellenlängen verschoben wird und/oder die Stabilität gegenüber Elektronen im fertigen OLED-Bauteil erhöht wird.

Im Folgenden sollen zur Erläuterung einige beispielhafte, geeignete Guanidinat-Liganden aufgezeigt werden:

Besonders bevorzugt ist das hpp-Anion selbst (nachfolgende mit 5a bezeichnet). Die Länge der beiden Brücken, die die Stickstoffatome miteinander verbinden können unabhängig voneinander variiert werden (nachfolgend mit 5b bezeichnet). Dabei sind n bzw. m ganze Zahlen, die unabhängig voneinander zwischen 1 und 10 gewählt werden können, wobei n und/oder m mit 2, 3 oder 4 bevorzugt sind. Die nachfolgend als 5c bezeichnete Struktur zeigt beispielhaft das verbrückte Guanidingrundgerüst mit n und m = 3. Die Substituenten R₁ - R₁₂ können gleich oder verschieden sein und haben hierbei die selbe Bedeutung wie die Substituenten R₁ - R₄, die vorstehend für die allgemeine Struktur des Guanidinat-Liganden definiert wurden. Im Unterschied dazu können in Struktur 5c auch jeweils zwei Substituenten zueinander benachbarter KohlenstoffAtome miteinander verbunden sein, und insbesondere Alkylen-Brücken darstellen, so dass ein, zwei oder mehrere Ringe ausgebildet werden, die unabhängig voneinander insbesondere 5-oder 6-gliedrig sein können.

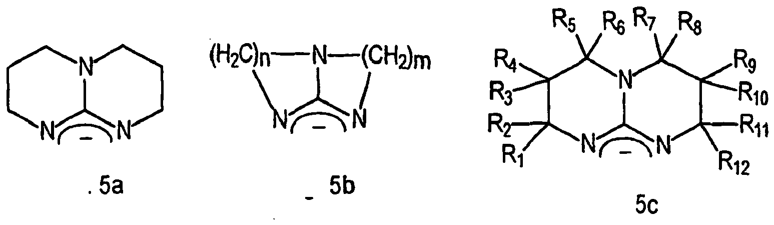

Weiterhin geeignet sind Verbindungen der nachfolgenden Strukturen 6a und 6b; sie zeigen ein Derivat mit kondensierten aromatischen Ringsystemen. Besonders bevorzugt ist hier n = 0, 1, 2 und m = 0, 1, 2. Die Substituenten R₁ bis R₄ in Struktur 6b können dabei können gleich oder verschieden sein und haben die selbe Bedeutung wie die Substituenten R₁ - R₄, die vorstehend für die allgemeine Struktur des Guanidinat-Liganden definiert wurden. Anders also dort definiert, können in Struktur 6b die Substituenten R₁ oder R₂ mit den Substituenten R₃ oder R₄ verbunden sein, und insbesondere eine Alkylen-Brücke darstellen, so dass ein Ring ausgebildet wird, der insbesondere 5- oder 6-gliedrig sein kann. Die Substituenten R₅ und R₆ in Struktur 6a und 6b stehen für ein komplettes Substitutionsmuster, das auch aus mehreren einzelnen Substituenten, die jeweils die vorstehende Bedeutung der Reste R₁ - R₄ haben können, aufgebaut sein kann.

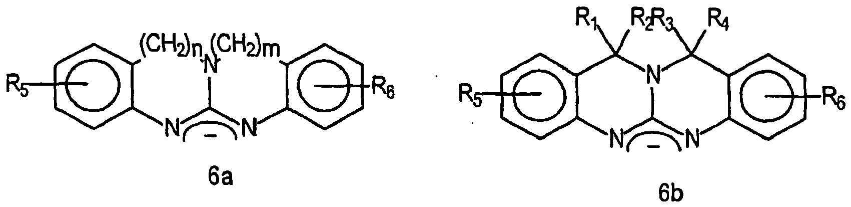

Weiterhin geeignet sind Verbindungen der nachfolgenden Strukturen 7a-c. Sie zeigen verschiedene Liganden mit zentralem Guanidinkern mit aromatischen Substituenten. Diese können dabei einzeln (7a), verbrückend - wobei sowohl Brücken zwischen verschiedenen aromatischen Substituenten (7b) als auch Brücken zwischen zwei an einen aromatischen Substituenten gebundenen Subsituenten Rₓ vorliegen können - oder kondensiert (7c) angeordnet sein. Rₓ (x = 1 - 4) können gleich oder verschieden sein und stellen dabei jeweils einen oder mehrere an einen Ring gebundene Substituenten, die wie vorstehend für die allgemeine Struktur des Guanidinat-Liganden definiert sind, dar.

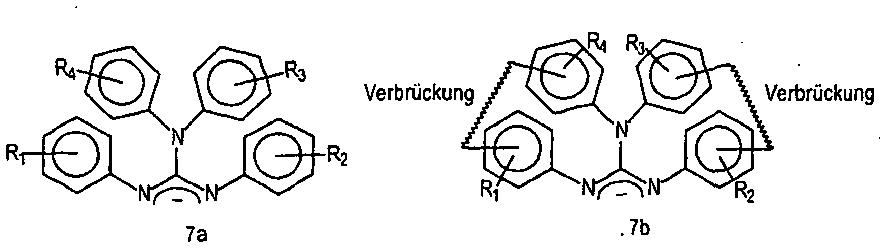

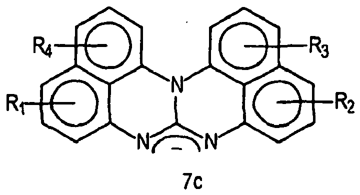

Ferner geeignet sind Verbindungen der nachfolgenden Strukturen 8a-8d und 9a-9h.Die Sturkuren 8a-8d stellen Guanidinderivate mit gesättigten Ringsystemen bzw. Substituenten dar. Rₓ (x = 1 - 4) können darin gleich oder verschieden sein und stellen dabei jeweils einen oder mehrere an einen Ring gebundene Substituenten, der wie vorstehend für die allgemeine Struktur des Guanidinat-Liganden definiert ist, dar.

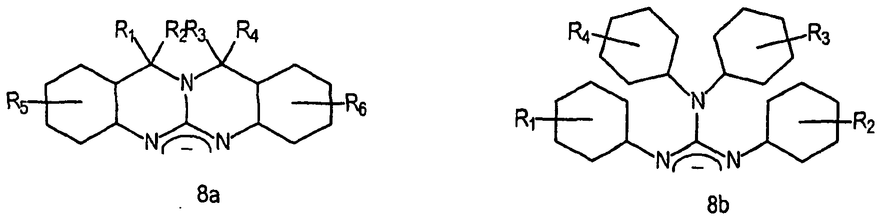

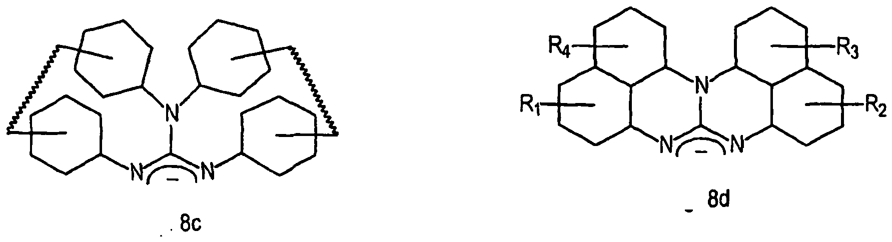

Die Sturkuren 9a-9h stellen gemischte bzw. höher kondensierte Varianten der Strukturen 7 a-d und 8 a-d dar.

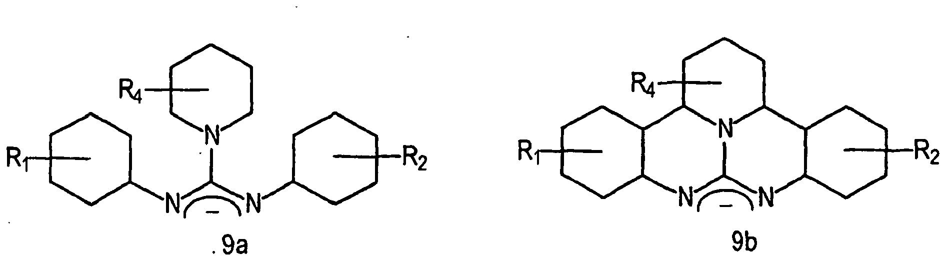

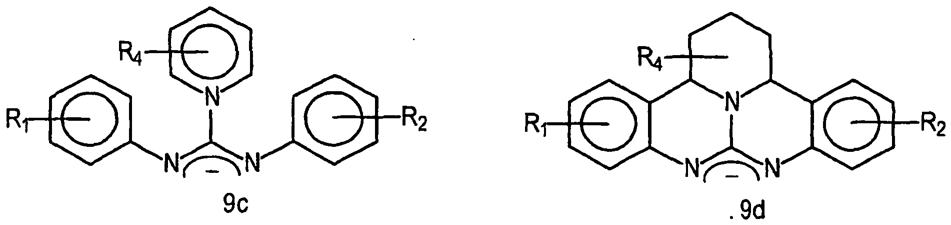

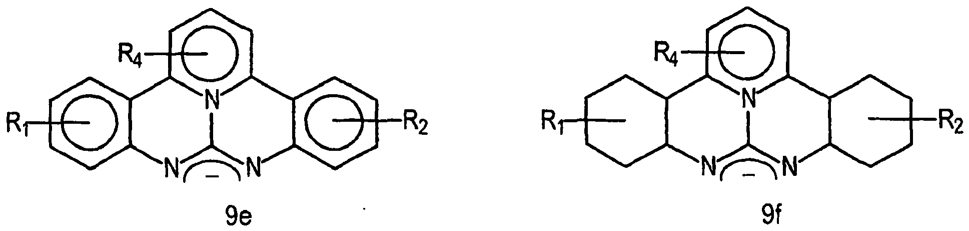

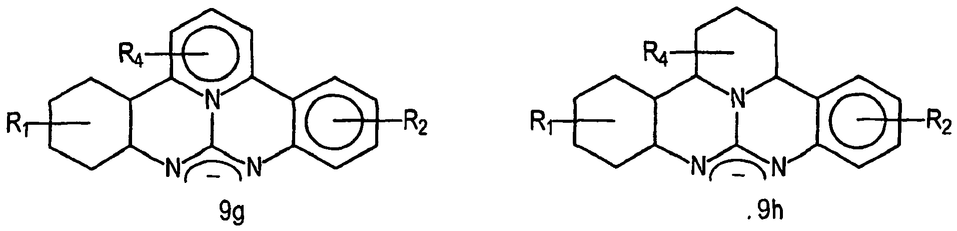

Im Sinne der Erfindung sind auch Guandinderivate, die weitere heterozyklische Substituenten (aromatisch oder aliphatisch) enthalten, geeignet. Nachfolgend sind einige Strukturformeln abgebildet, die Guandinderivate mit ankondensierten sechsgliederigen Ringen zeigen. Im Falle.aromatischer Ringe können X₁-X₁₀ unabhängig voneinander C-H bzw. C-R oder N sein. Im Falle aliphatischer Ringe können X₁-X₁₀ unabhängig voneinander CH₂, -C-HR bzw. C-R₁R₂ oder N bzw. NH oder NR sein. Die Reste R bzw. R₁ bzw. R₂ können hierbei jeweils gleich oder verschieden sein und stellen Substituenten, die wie vorstehend für die allgemeine Struktur des Guanidinat-Liganden definiert sind, dar. 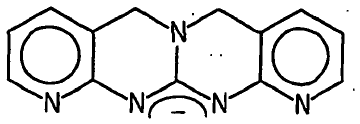 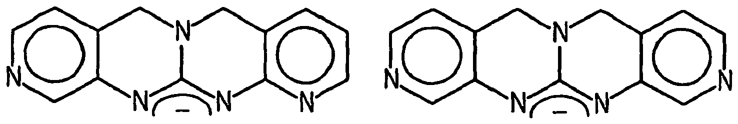 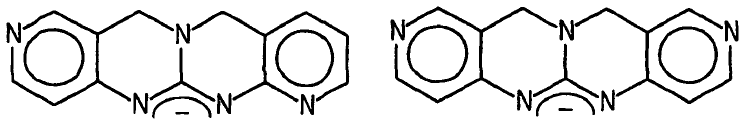 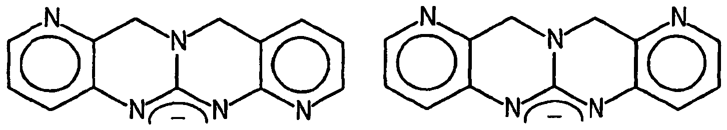 beziehungsweise 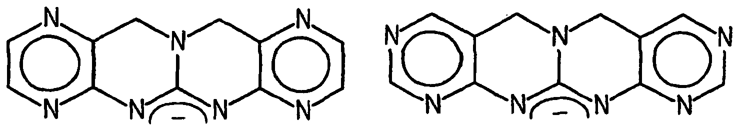 allgemein

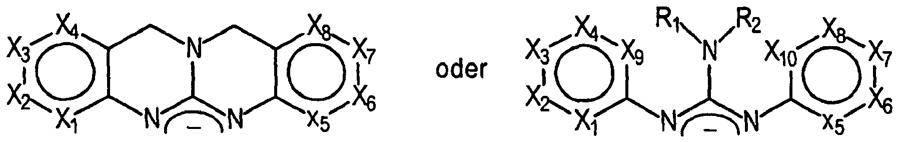

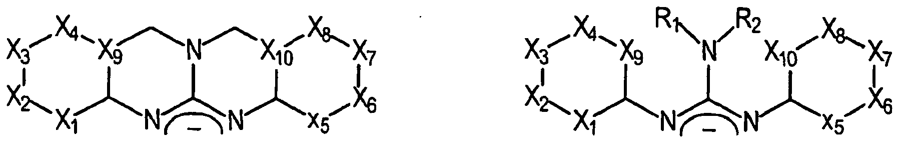

In analoger Weise sind auch Vertreter der vorstehenden Guandinderivate mit ankondensierten Chinolin- und Isochinolin-Gruppen geeignet. Weiterhin geeignet sind Guandinderivate, bei denen die vorstehend gezeigten aromatischen Ringe auch hydriert sind und/oder mit einem bzw. mehreren Substituenten R substituiert sind (Die Reste R können hierbei gleich oder verschieden sein und stellen Substituenten, die wie vorstehend für die allgemeine Struktur des Guanidinat-Liganden definiert sind, dar).

Schließlich sind auch Guanidinderivate der nachfolgenden Strukturen (mit Imidazol bzw. Benzimidazolsubstituenten) geeignet. Die Substituenten R₁-R₈ können hierbei gleich oder verschieden sein und stellen Substituenten, die wie vorstehend für die Guandin-Anion-Gruppe definiert sind, dar. Ist jedoch wenigstens R₁ oder R₂ = H besteht die Möglichkeit durch eine weitere Deprotonierung zweifach negativ geladene Ligandensysteme zu erhalten. In analoger weise sich auch Guanidinderivate mit Pyrazol-Gruppen geeignet.

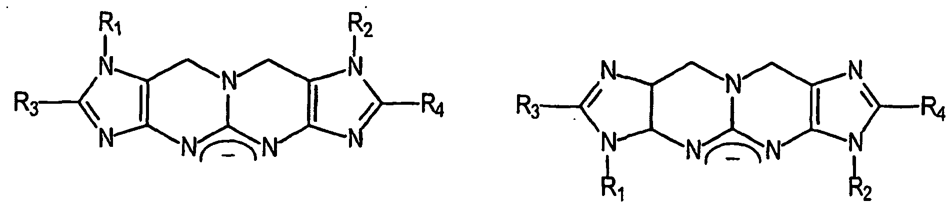

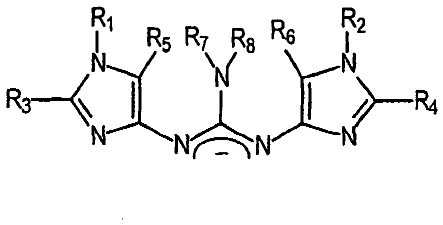

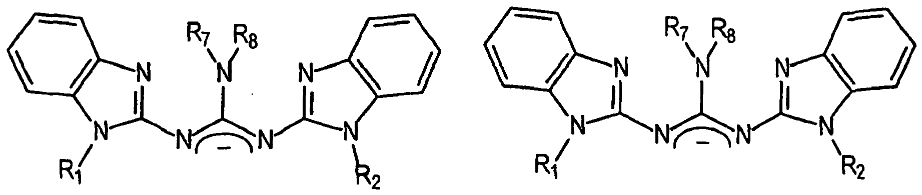

Des Weiteren sind im Sinne der Erfindung Liganden mit Guanidingrundgerüst geeignet, die zusätzlich P, S, O, As, Sb, F, oder auch metallorganische Substituenten, wie bespielweise Ferrocenyl, Phthalocyaninyl (wobei das Zentralatom Zn, Fe, Ni etc. sein kann) enthalten.

Mit der Erfindung gelingt erstmals die Identifikation eines Strukturelements, das zur Erhöhung der Stabilität in metallorganischen phosphoreszenten Emittern, insbesondere in Triplettemittern herangezogen werden kann. Die erfindungsgemäßen Liganden beinhalten das Anion eines Guanidin-Derivates, das in verschiedenartiger Weise an Metallatome koordiniert werden kann. Die anionischen Liganden werden dabei durch Deprotonierung des entsprechenden neutralen Liganden mit Guandineinheit erhalten. Die Ligandsysteme können dabei weitere Substituenten enthalten, die die Koordination an weitere Metallatome fortsetzen.

Die Erfindung wird nachfolgend noch anhand von Figuren und Ausführungsbeispielen näher erläutert:
Figur 1 zeigt die schematische Seitenansicht eines strahlungsemittierenden Bauelements.
Figur 2a bis c zeigt Photolumineszenzspektren für verschiedene Metallkomplexverbindungen.

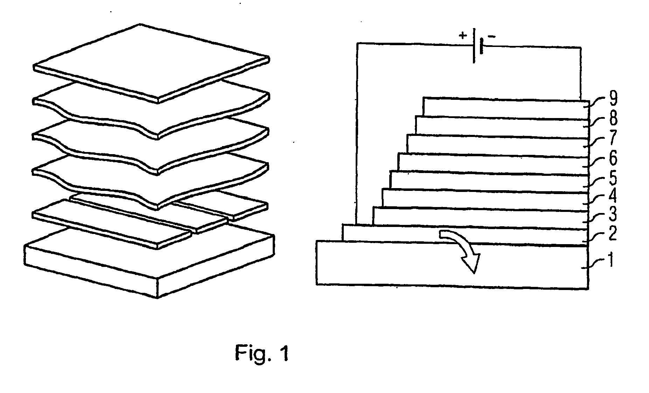

Die Figur 1 zeigt den schematisierten Schichtaufbau eines organischen selbstemittierenden Bauteils. Von unten nach oben ist folgender Schichtaufbau realisiert:

Ganz unten befindet sich das Substrat 1, das beispielsweise transparent sein kann und aus Glas sein kann. Darauf befindet sich die untere Elektrodenschicht 2, die beispielsweise ein transparentes leitendes Oxid wie beispielsweise Zinkoxid, zinnoxid, Cadmiumoxid, Titanoxid, Indiumoxid oder Indiumzinnoxid (ITO) sein kann.. Über dieser Elektrodenschicht 2 liegt die Lochinjektionsschicht 3 , über der wiederum die Lochtransportschicht 4 angeordnet ist und über der die organische aktive Schicht, die Emissionsschicht 5 liegt. Auf der Emissionsschicht 5 liegt die Lochblockierende Schicht 6, auf der die Elektronentransportschicht 7 und schließlich die Elektroneninjektionsschicht 8 mit angrenzender oberer Elektrode 9, beispielsweise einer Metallelektrode oder einer weiteren transparenten Elektrode, z.B. aus einem der vorstehend genannten transparenten leitenden Oxide.

Bei Anlegen einer Spannung zwischen oberer und unterer Elektrode fließt Strom durch das Bauteil und in der organischen aktiven Schicht 5 werden Photonen freigesetzt, die in Form von Licht über die untere Elektrode 2 und das Substrat 1 das Bauteil verlassen.

In der Emissionsschicht 5 sind erfindungsgemäß in einer Matrix Metallkomplexe vorgesehen, die ein Ionisierungspotential kleiner/gleich 5 eV haben. Insbesondere sind die erfindungsgemäßen Metallkomplexe vorgesehen, die zumindest einen Liganden, der über eine Guanidin-Anion-Gruppe am Zentralatom koordiniert, aufweisen.

Die Herstellung eines derartigen strahlungsemittierenden Bauteils kann z.B. folgendermaßen erfolgen:

Mittels HF-Sputtern wird zunächst eine ITO-Schicht als Anode auf einer Glasplatte abgeschieden. Zur Abscheidung der weiteren funktionellen Schichten wird dieses Substrat in einen Rezepienten eingebracht; dieser enthält eine oder mehrere Quellen, in denen organisches Material (zur Herstellung der einzelnen funktionellen Schichten der strahlungsemittierenden Vorrichtung wie Emittermaterialien oder p- oder n-Dotanten) verdampft werden kann. Ferner werden ein oder mehrere Quellen für die Zuführung von einem oder mehreren verschiedenen Matrixmaterialien vorgesehen. Zur Ausbildung einer Lochinjektionsschicht wird aus einer Quelle mit Matrixmaterial und einer Quelle mit p-Dotant gemeinsam auf der Glasplatte mit der Anode abgeschieden. Entsprechend erfolgt die gemeinsame Abscheidung von Dotant und Matrixmaterial für die Lochtransportschicht. Anschließend erfolgt die gemeinsame Abscheidung eines Matrixmaterials und des erfindungsgemäßen Metallkomplexes und gegebenenfalls einer weiteren phosphoreszierenden Verbindung, wobei die Emitterschicht erhalten wird. Die Abscheidung weiterer enthaltener Schichten wie Blockierschicht, Elektronentransportschicht und Elektroneninjektionsschicht erfolgt analog. Abschließend wird eine 150 nm dicke Aluminiumschicht als reflektive Elektrode gebildet.

Im Folgenden werden Ausführungsbeispiele zur Herstellung von Übergangskomplexverbindungen angegeben.

Guanidinderivate können beispielsweise nach den Herstellungsverfahren gemäß Dalton Trans., 2006, 4623-4631, Inorganic Chemistry, 2006, 45, 5493-5500 sowie Inorg. Chem 1997, 36, 867 und gemäß der WO 2005/086251 A2, der US 4,797,487 und der EP 0 198 680 A1 synthetisiert werden. Für diese Synthesen benötigte Triamine sind z.B. gemäß FI 82445 erhältlich. Auf diese Herstellungsverfahren wird hiermit vollinhaltlich Bezug genommen.

### 1. Allgemeine Vorschrift zur Herstellung bicyclischer Guanidinderivate:

100 mmol Schwefelkohlenstoff werden zu einer Lösung von 100 mmol Triamin in 150 ml p-Xylol zugefügt. Das erhaltene Gemisch wird anschließend so lange unter Rückfluss erhitzt, bis kein Schwefelwasserstoff mehr gebildet wird (ca. 10 Tage). Das bicyclische Guanidin kann mittels Kristallisation durch Abkühlen der Xylol-Lösung und üblicherweise auch durch Sublimation gereinigt werden.
Die Synthese wird nachfolgend anhand eines substituierten oder unsubstituerten N-3-Aminopropyl-1,3-propandiamins gezeigt:

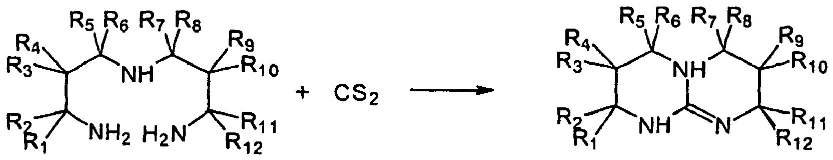

### 2. Allgemeine Vorschrift zur Herstellung monocyclischer oder acyclischer Guanidinderivate:

100 mmol des Thioharnstoff-Derivats werden zu einer Lösung von 100 mmol primärem Amin in 150 ml p-Xylol zugefügt. Das erhaltene Gemisch wird anschließend so lange unter Rückfluss erhitzt, bis kein Schwefelwasserstoff mehr gebildet wird (ca. 10 Tage). Das erhaltene Guanidin-Derivat kann mittels Kristallisation durch Abkühlen der Xylol-Lösung und üblicherweise auch durch Sublimation gereinigt werden:

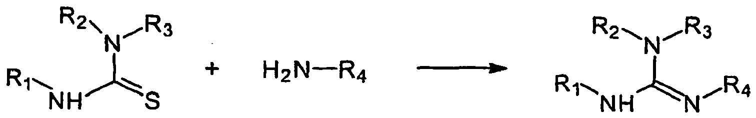

### 3. Allgemeine Vorschrift zur Herstellung von Komplexen mit Guanidiat-Liganden:

1 mmol eines Metallsalzes der Formel L¹ₖL²ₘMXₙ oder der Formel [L¹ₖL²ₘ₋₁MXₙ]₂ werden in 20 ml Dichlormethan oder in 20 ml Tetrahydrofuran suspendiert und auf -70°C abgekühlt. Ferner wird jeweils n*1 mmol Natriummethylat (oder alternativ Butyllithium) und n*1 mmol Guanidinderivat in 40 ml Dichlormethan suspendiert (hierbei entspricht n der Anzahl zu koordinierender Guanidinat-Liganden) und ebenfalls auf -70°C gekühlt. Diese Suspension wird langsam zur Suspension des Metallsalzes zugetropft. Die Reaktionsmischung wird 48h bei Raumtemperatur gerührt. Anschließend wird über eine Fritte filtriert und mit Dichlormethan nachgewaschen. Das Filtrat wird eingeengt und im Vakuum getrocknet. Gegebenenfalls kann der erhaltene Guanidinat-Komplex durch Waschen mit Pentan gereinigt werden.

Bei der angegebenen Formel des Metallkomplexes entspricht L¹ einem beliebigen Liganden; k entspricht der Anzahl pro Metall koordinierter L¹-Liganden und kann auch 0 sein; L² entspricht einem beliebigen Neutralliganden, der bei der Umsetzung mit dem Guanidinat-Liganden abgespalten wird (z.B. ein C₂H₄-Ligand); m entspricht der Anzahl pro Metall koordinierter L²-Liganden und ist > 0, für einzähnige Neutralliganden (wie z.B. C₂H₄) gilt m-1=n; X ist ein Halogen-Ligand oder ein anderer einfach negativ geladener Ligand (z.B. ein Carboxylat-Ligand wie Acetat); n entspricht der Anzahl zu koordinierender Guanidinat-Liganden bzw. der Anzahl der Liganden X;

### 4. Synthese von Di(µ-chloro)-bis[(phenyl-pyridino)platin(II)] = Verbindung 1 (Vergleichsbeispiel)

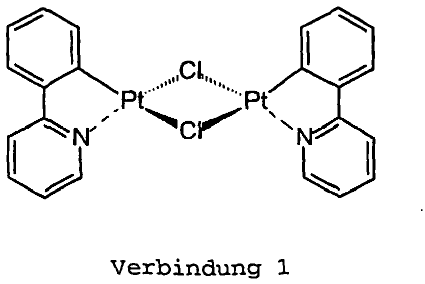

12 mmol (4,98 g) Kaliumtetrachloroplatinat werden in 24 ml heißem entgastem Wasser gelöst und unter starkem Rühren wieder abgekühlt. Dabei fällt das Kaliumtetrachloroplatinat als feine Suspension aus. Zu dieser Suspension wird eine Lösung von 12 mmol (1,86 g) Phenylpyridin in 72 ml Ethoxyethanol zugetropft. Die Suspension wird auf 70°C erhitzt, wobei sich zunehmend ein dunkelgrüner Niederschlag bildet. Die Suspension wird zum Ausfällen des Rohprodukts mit 30 ml Wasser unterschichtet und nach ca. 2h umgerührt. Das Rohprodukt wird abgesaugt und mehrmals mit einem Wasser/Alkohol-Gemisch (10:1) gewaschen. An dieser Stelle wird das Produkt luftstabil. Anschließend wird es im Vakuum ca. 20h getrocknet. Verschiedene Chargen zeigen im Feststoff eine gelbe bis grüne Färbung je nach Anteil an Verunreinigungen. Das Rohprodukt kann jedoch ohne weitere Reinigung für die folgenden Versuche verwendet werden.
Ausbeute: : 3,56 g (77,2%)

### 5. Synthese von Di(µ-chloro)-bis[(2,4-difluor-phenyl-pyridino)platin(II)] = Verbindung 2 (Vergleichsbeispiel)

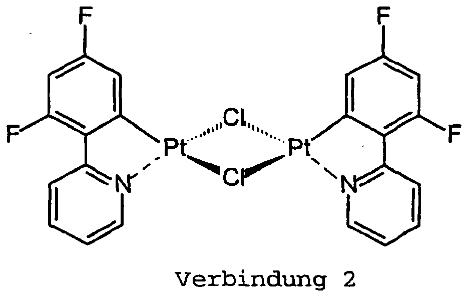

7,23 mmol (3 g) Kaliumtetrachloroplatinat werden in 14 ml heißem entgastem Wasser gelöst und unter starkem Rühren auf 30°C abgekühlt. Dabei fällt das Kaliumtetrachloroplatinat als feine Suspension aus. Zu dieser Suspension wird eine Lösung von 7,23 mmol (1,387g) 2,4-Difluor-phenylpyridin in 42 ml Ethoxyethanol langsam zugetropft. Die Suspension wird ca. 20h auf 70°C erhitzt, wobei sich zunehmend ein gelb-grüner Niederschlag bildet. Die Suspension wird nach Abkühlen auf Raumtemperatur zum Ausfällen des Rohprodukts mit 30 ml Wasser unterschichtet und nach ca. 2h umgerührt. Das gelb-grüne Rohprodukt wird abgesaugt und mehrmals mit einem Wasser/AlkoholGemisch (10:1) gewaschen. Im Exsikkator unter Vakuum ca. 20h trocknen.
Ausbeute: 2,36g (78%)

### 6. Synthese von Di(µ-chloro)-bis[(di-pyridylamino)platin(II)] = Verbindung 3 (Vergleichsbeispiel)

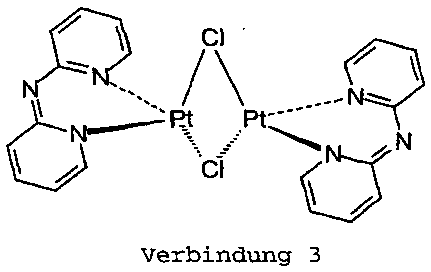

3 mmol (1,245 g) Kaliumtetrachloroplatinat werden in 6 ml heißem entgastem Wasser gelöst und unter starkem Rühren auf 30°C abgekühlt. Dabei fällt das Kaliumtetrachloroplatinat als feine Suspension aus. Zu dieser Suspension wird eine Lösung von 3 mmol (0,514 g) Dipyridylamin in 45 ml Ethoxyethanol langsam zugetropft. Die Suspension wird ca. 20h auf 70°C erhitzt, wobei sich zunehmend ein cremefarbener Niederschlag bildet. Die Suspension wird nach Abkühlen auf Raumtemperatur zum Ausfällen des Rohprodukts mit 40 ml Wasser unterschichtet und nach ca. 2h umgerührt. Das Rohprodukt wird abgesaugt und mehrmals mit einem Wasser/Alkohol-Gemisch (10:1) gewaschen. Im Exsikkator unter Vakuum ca. 20h trocknen.
Ausbeute: 1 g (83%)

### 7. Synthese von Di(µ-hpp)-bis[(phenyl-pyridino)platin(II)] = Verbindung 4

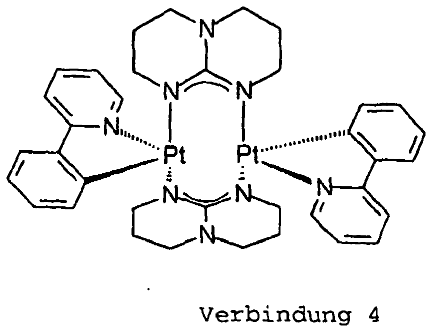

0,39 mmol (0,3 g) Di(µ-chloro)-bis[(phenyl-pyridino)platin(II)] (Verbindung 1) werden in 25 ml Dichlormethan suspendiert. Gleichzeitig werden 0,78 mmol (108,6 mg) Hhpp und 0,78 mmol (42,13 mg) Natriummethylat in 20 ml Dichlormethan suspendiert. Beide Suspensionen werden unter dem Rühren auf -70°C abgekühlt und dann Hhpp-Suspension wird zu der Di(µ-chloro)-bis[(phenyl-pyridino)platin(II)]-Suspension zugegeben. Die Mischung wird ca. 48h bei Raumtemperatur gerührt. Nach 48h wird das Gemisch über eine P4-Fritte abfiltriert und mit Dichlormethan mehrmals nachgewaschen. Die Lösung wird im Vakuum eingeengt. Anschließend wird die Substanz mit Pentan gewaschen. Die Pentan-Extraktion zeigt aber im Photolumineszenzspektrum das gleiche Ergebnis, wie das gewaschene Produkt.
Ausbeute: praktisch quantitativ

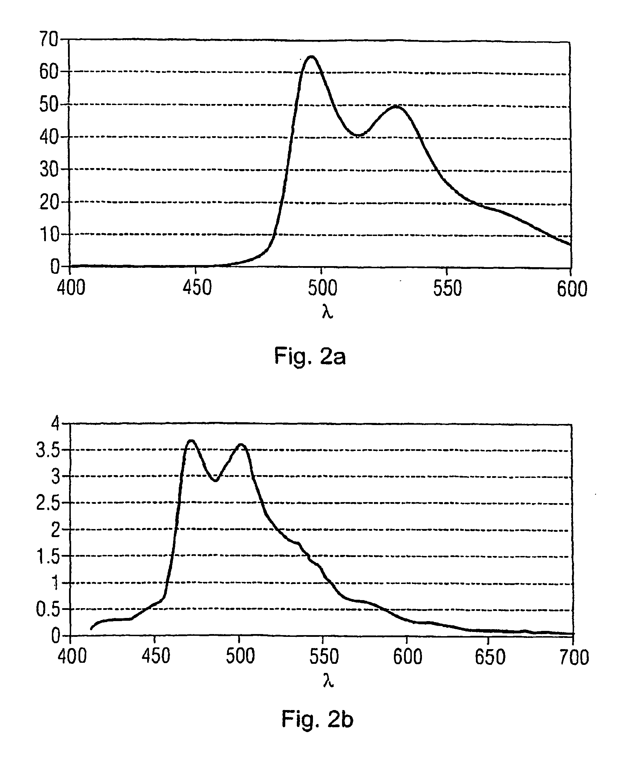

In Figur 2a ist das Photolumineszenzspektrum der Verbindung 8 gezeigt, mit einem Emissionsmaximum bei 498 nm und 531 nm.

### 8. Synthese von Di(µ-hpp)-bis[(2,4-difluor-phenyl-pyridino)platin(II)] = Verbindung 5

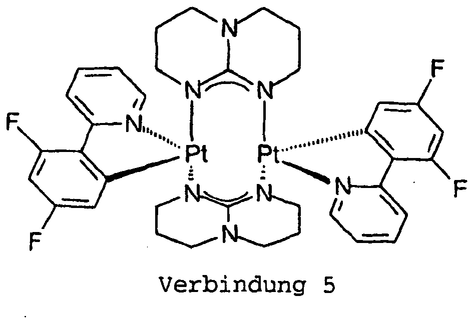

1,19 mmol (1 g) Di(µ-chloro)-bis[(2,4-difluor-phenyl-pyridino)platin(II)] (Verbindung 2) werden in 20 ml Dichlormethan suspendiert und auf -70°C abgekühlt. Dazu wird eine Mischung aus 2,377 mmol (128,4 mg) Natriummethylat und 2,377 mmol (330,9 mg) Hhpp, suspendiert in 40 ml Dichlormethan und ebenfalls auf -70°C gekühlt, langsam zugetropft. Die grünliche Reaktionsmischung wird 48h bei Raumtemperatur gerührt, wobei sich die Mischung bräunlich färbt. Anschließend wird über eine Fritte filtriert und mit Dichlormethan.nachgewaschen. Das Filtrat wird eingeengt, man erhält ein bräunlichbeiges Produkt. Eine mit Ether herausgelöste Fraktion ergibt das gleiche PL-Spektrum wie das Rohprodukt.
Ausbeute: praktisch quantitativ

In Figur 2b ist das Photolumineszenzspektrum der Verbindung 9 gezeigt, mit einem Emissionsmaximum bei 473 nm und 501 nm.

### 9. Synthese von Di(µ-hpp)-bis[(dipyridylamino)platin(II)] = Verbindung 6

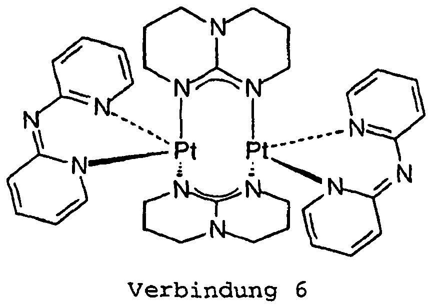

1,25 mmol (1 g) Di(µ-chloro)-bis[(di-pyridylamino)platin(II)] (Verbindung 3) werden in 10 ml Dichlormethan suspendiert und auf -70°C abgekühlt. Dazu wird eine Mischung aus 2,496 mmol (134,8 mg) Natriummethylat und 2,496 mmol (347,4 mg) Hhpp, suspendiert in 35 ml Dichlormethan und ebenfalls auf -70°C gekühlt, langsam zugetropft. Die Reaktionsmischung färbt sich dabei gelb. Man lässt 48h unter Rühren bei Raumtemperatur reagieren. Danach wird die Substanz durch die P4-Fritte abfiltriert und mit Dichlormethan mehrmals nachgewaschen. Das Filtrat wird eingeengt und im Vakuum getrocknet.
Ausbeute 1,04 g (83%)

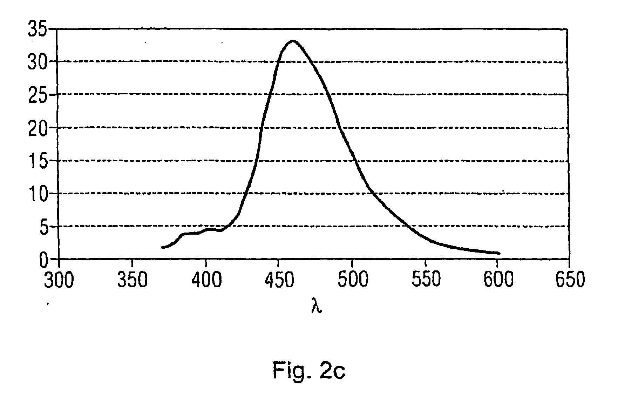

In Figur 2c ist das Photolumineszenzspektrum der Verbindung 10 gezeigt, mit einem Emissionsmaximum bei 463 nm.

## Patentansprüche

1. Strahlungsemittierendes organisches Bauteil, umfassend ein Substrat (1), zumindest eine untere Elektrodenschicht (2), zumindest eine organische strahlungsemittierende Schicht (5) und eine darüber angeordnete obere Elektrodenschicht (9), wobei die strahlungsemittierende Schicht (5) eine Matrix aufweist, in der zumindest ein strahlungsemittierender Metallkomplex enthalten ist, der zumindest einen substituierten oder unsubstituierten Guanidinat-Liganden mit einer Guanidin-Anion-Gruppe aufweist, wobei die Guanidin-Anion-Gruppe an das im Metallkomplex enthaltene Zentralatom zweifach koordiniert ist oder in einem zumindest zweikernigen Metallkomplex die zumindest zwei Metallatome verbrückt.

2. Bauteil nach Anspruch 1, wobei das Bauteil jeweils zwischen den Elektrodenschichten neben der strahlungsemittierenden Schicht (5) noch eine oder mehrere Hilfsschichten hat.

3. Bauelement nach Anspruche 1 oder 2, wobei das Substrat (1) und die untere Elektrodenschicht (2) transparent sind.

4. Bauteil nach einem der Ansprüche 1 bis 3, wobei der in der strahlungsemittierenden Schicht (5) enthaltene Metallkomplex zumindest ein Zentralatom, ausgewählt aus der Gruppe der Übergangsmetalle und der Lanthanoide, aufweist.

5. Bauteil nach einem der Ansprüche 1 bis 4, wobei der in der strahlungsemittierenden Schicht (5) enhaltene Metallkomplex als Guanidinat-Liganden zumindest einen hpp-Liganden aufweist.

6. Bauteil nach einem der vorhergehenden Ansprüche, wobei zumindest zwei der Stickstoffatome des Guanidinat-Liganden durch Kohlenwasserstoffbrücken, die substituiert oder unsubstituiert und/oder aromatisch, kondensiert aromatisch oder nicht aromatisch und/oder heterocyclisch sind, verbrückt sind.

7. Bauteil nach einem der vorhergehenden Ansprüche, wobei der Guanidinat-Ligand bicyclisch ist und das bicyclische System die N-Atome des Guanidinat-Liganden enthält.

8. Bauteil nach einem der vorhergehenden Ansprüche, wobei der in der Emissionsschicht enthaltene Metallkomplex homoleptisch ist.

9. Bauteil nach einem der vorhergehenden Ansprüche, das tiefblau, hellblau und/oder blaugrün emittiert.

10. Phosphoreszente Metallkomplexverbindung, die zumindest ein metallisches Zentralatom M, das aus einer Gruppe ausgewählt ist, die Ir, Pt, Au, Re, Ru, Os, Pd, Ag und Lanthanoide umfasst, und zumindest einen durch das metallische Zentralatom M koordinierten substituierten oder unsubstituierten Guanidinat-Liganden mit einer Guanidin-Anion-Gruppe umfasst sowie mindestens einen weiteren Liganden (Koliganden), wobei der mindestens eine Koligand über zumindest ein C-, N-, P-, As-, Sb-, O-, S- und/oder Se-Atom an das Zentralatom M koordiniert ist wobei die Guanidin-Anion-Gruppe an das im Metallkomplex enthaltene Zentralatom zweifach koordiniert ist oder in einem zumindest zweikernigen Metallkomplex die zumindest zwei Metallatome verbrückt.

11. Verbindung nach dem vorhergehenden Anspruch, wobei der Guanidinat-Ligand bicyclisch ist und das bicyclische System die N-Atome des Guanidinat-Liganden enthält.

12. Verbindung nach einem der zwei vorhergehenden Ansprüche, wobei zumindest ein Koligand zwei- oder mehrzähnig ist.

## Claims

1. Radiation-emitting organic component comprising a substrate (1), at least one lower electrode layer (2), at least one organic radiation-emitting layer (5) and an upper electrode layer (9) arranged above it, said radiation-emitting layer (5) having a matrix in which at least one radiation-emitting metal complex is present and has at least one substituted or unsubstituted quanidinate ligand with a guanidine anion group, said guanidine anion group being doubly coordinated to the central atom present in the metal complex or bridging the at least two metal atoms in an at least dinuclear metal complex.

2. Component according to Claim 1, wherein the component in each case also has one or more auxiliary layers as well as the radiation-emitting layer (5) between the electrode layers.

3. Component according to Claim 1 or 2, wherein the substrate (1) and the lower electrode layer (2) are transparent.

4. Component according to any of Claims 1 to 3, wherein the metal complex present in the radiation-emitting layer (5) has at least one central atom selected from the group of the transition metals and the lanthanoids.

5. Component according to any of Claims 1 to 4, wherein the metal complex present in the radiation-emitting layer (5) has at least one hpp ligand as the guanidinate ligand.

6. Component according to any of the preceding claims, wherein at least two of the nitrogen atoms of the guanidinate ligand are bridged by hydrocarbon bridges which are substituted or unsubstituted and/or aromatic, fused aromatic or nonaromatic and/or heterocyclic.

7. Component according to any of the preceding claims, wherein the guanidinate ligand is bicyclic and the bicyclic system contains the nitrogen atoms of the guanidinate ligand.

8. Component according to any of the preceding claims, wherein the metal complex present in the emission layer is homoleptic.

9. Component according to any of the preceding claims, which emits in the deep blue, light blue and/or blue-green.

10. Phosphorescent metal complex which comprises at least one metallic central atom M selected from a group comprising Ir, Pt, Au, Re, Ru, Os, Pd, Ag and lanthanoids, and at least one substituted or unsubstituted guanidinate ligand which has a guanidine anion group and is coordinated by the metallic central atom M, and at least one further ligand (coligand), said at least one coligand being coordinated to the central atom M via at least one C, N, P, As, Sb, O, S and/or Se atom, said guanidine anion group being doubly coordinated to the central atom present in the metal complex or the at least two metal atoms being bridged in an at least dinuclear metal complex.

11. Complex according to the preceding claim, wherein the guanidinate ligand is bicyclic and the bicyclic system contains the nitrogen atoms of the guanidinate ligand.

12. Complex according to either of the two preceding claims, wherein at least one coligand is bi- or polydentate.

## Revendications

1. Élément organique émettant un rayonnement, comprenant un substrat (1), au moins une couche d'électrode inférieure (2), au moins une couche organique émettant un rayonnement (5) et une couche d'électrode supérieure (9) placée au-dessus, la couche émettant un rayonnement (5) comprenant une matrice qui contient au moins un complexe métallique émettant un rayonnement qui comprend au moins un ligand guanidinate substitué ou non substitué contenant un groupe anion guanidine, le groupe anion guanidine étant coordonné deux fois à l'atome central contenu dans le complexe métallique ou reliant les deux atomes métalliques ou plus dans un complexe métallique au moins binucléaire.

2. Élément selon la revendication 1, dans lequel l'élément comprend également une ou plusieurs couches auxiliaires à chaque fois entre les couches d'électrodes en plus de la couche émettant un rayonnement (5).

3. Élément selon la revendication 1 ou 2, dans lequel le substrat (1) et la couche d'électrode inférieure (2) sont transparents.

4. Élément selon l'une quelconque des revendications 1 à 3, dans lequel le complexe métallique contenu dans la couche émettant un rayonnement (5) comprend au moins un atome central, choisi dans le groupe des métaux de transition et des lanthanides.

5. Élément selon l'une quelconque des revendications 1 à 4, dans lequel le complexe métallique contenu dans la couche émettant un rayonnement (5) comprend au moins un ligand hpp en tant que ligand guanidinate.

6. Élément selon l'une quelconque des revendications précédentes, dans lequel au moins deux des atomes d'azote du ligand guanidinate sont reliés par des ponts hydrocarbonés, qui sont substitués ou non substitués et/ou aromatiques, aromatiques condensés ou non aromatiques et/ou hétérocycliques.

7. Élément selon l'une quelconque des revendications précédentes, dans lequel le ligand guanidinate est bicyclique et le système bicyclique contient les atomes N du ligand guanidinate.

8. Élément selon l'une quelconque des revendications précédentes, dans lequel le complexe métallique contenu dans la couche d'émission est homoleptique.

9. Élément selon l'une quelconque des revendications précédentes, qui émet dans le bleu foncé, le bleu clair et/ou le bleu-vert.

10. Composé complexe métallique phosphorescent, qui comprend au moins un atome central métallique M, qui est choisi dans un groupe comprenant Ir, Pt, Au, Re, Ru, Os, Pd, Ag et les lanthanides, et au moins un ligand guanidinate substitué ou non substitué contenant un groupe anion guanidine coordonné par l'atome central métallique M, ainsi qu'au moins un ligand supplémentaire (co-ligand), le ou les co-ligands étant coordonnés à l'atome central M par au moins un atome C, N, P, As, Sb, O, S et/ou Se, le groupe anion guanidine étant coordonné deux fois à l'atome central M contenu dans le complexe métallique ou reliant les deux atomes métalliques ou plus dans un complexe métallique au moins binucléaire.

11. Composé selon la revendication précédente, dans lequel le ligand guanidinate est bicyclique et le système bicyclique contient les atomes N du ligand guanidinate.

12. Composé selon l'une quelconque des deux revendications précédentes, dans lequel au moins un co-ligand est bi- ou polydentate.
